(19) Europäisches Patentamt

European Patent Office

Office européen des brevets

(11) Publication number: **0 482 467 A2**

# EUROPEAN PATENT APPLICATION

(12)

(21) Application number: **91117516.4**

(22) Date of filing: **14.10.91**

(51) Int. Cl.⁵: **A61L 25/00**

(30) Priority: **15.10.90 JP 273354/90**

(43) Date of publication of application:
**29.04.92 Bulletin 92/18**

(84) Designated Contracting States:
**DE FR GB**

(71) Applicant: **NISSHINBO INDUSTRIES, INC.**
**3-10, Nihonbashi Yokoyama-cho**
**Chuo-ku Tokyo 103(JP)**

(72) Inventor: **Ikada, Yoshita**
**2-182, Gokasho Hirookadani**
**Uji-shi, Kyoto(JP)**
Inventor: **Morimoto, Kiyotake**
**3552, Koshigaya**
**Koshigaya-shi, Saitama-ken(JP)**
Inventor: **Takagi, Yasushi**
**3-13-103, Nishiarai Hon-cho 5-chome,**
**Adachi-ku**
**Tokyo(JP)**

(74) Representative: **Kraus, Walter, Dr. et al**
**Patentanwälte Kraus, Weisert & Partner**
**Thomas-Wimmer-Ring 15**
**W-8000 München 22(DE)**

(54) **Surgical adhesive.**

(57) A surgical adhesive comprising
(a) 100 parts by weight of an aliphatic isocyanate-terminated urethane prepolymer, and
(b) 0.1-5 parts by weight of a metal carboxylate as a curing catalyst.

EP 0 482 467 A2

The present invention relates to a surgical adhesive. More particularly, the present invention relates to a surgical adhesive comprising an aliphatic isocyanate-terminated urethane prepolymer as a base.

A cyanoacrylate type adhesive and a fibrin paste are known as surgical adhesives which are in actual use clinically. The cyanoacrylate type adhesive is characterized in that it invites polymerization in the presence of a very small amount of water and completes curing rapidly. However, the adhesive has various drawbacks; for example, the adhesive is very fragile and has no flexibility when cured, forms highly toxic formaldehyde when hydrolyzed, and is difficult to handle because of high fluidity. The fibrin paste, which is derived from living bodies, has drawbacks in that it may invite infection with serum hepatitis and is significantly inferior to the cyanoacrylate adhesive in adhesivity and adhesion speed.

Hence, studies on development of new medical elastic adhesive were made recently. For example, there was proposed an elastic adhesive comprising a reactive liquid urethane prepolymer having a structure of a hydrophilic polyol whose two ends have been capped with a diisocyanate (reference is made to Japanese Laid-Open Patent publication No. 148666/1987 and Japanese Laid-Open Patent Publication No. 290465/1987). This urethane type adhesive uses, as the isocyanate component an aromatic diisocyanate such as diphenylmethane diisocyanate (MDI), tolylene diisocyanate (TDI) or the like. An aromatic isocyanate, when taken into a living body, is difficult to methabolize and, when decomposed, produces an aromatic amine which may be carcinogenic; therefore, it is not a preferable material. In fact, it is reported that when an aromatic isocyanate-based polyurethane is introduced into a living body for an application such as implanting material, a low-molecular compound having high toxicity appears as a dissolution product or a decomposition product [reference is made to Chris Batich, Jerry Williams and Roy King, Applied Biomaterials, Vol. 23, No. A3, 311-319 (1989)].

Meanwhile, in order to make it possible to obtain a cured material rapidly according to a prepolymer method, it is necessary to prepare a prepolymer having a functional group of sufficiently high reactivity. In general, a prepolymer has low molecular mobility and accordingly the reaction between the functional groups is not easy. Further, an aliphatic isocyanate group has lower reactivity than an aromatic isocyanate group. Accordingly, the use of an aliphatic isocyanate in place of an aromatic isocyanate in the curing step by a prepolymer method makes rapid curing difficult (reference is made to Takehisa Matsuda et al., ARTIFICIAL ORGAN, Vol. 15, No. 1, p. 174, 1986).

Aliphatic polyesters typified by polyglycolic acid, polylactic acid, etc. are easily hydrolyzed in the presence of water to form hydrolysis products which can be metabolized; accordingly, said polyesters are known as a medical material which can be decomposed and absorbed in a living body. Also, in a surgical adhesive, there is desired a type which can sustain its action until the damaged tissue of living body is recovered by the self restorability of the tissue, and thereafter can be rapidly decomposed and metabolized. In the surgical adhesive disclosed in the above Japanese Laid-Open Patent Publication Nos. 148666/1987 and 290465/1987, if an ester bond is introduced into the main chain of the aromatic isocyanate-terminated prepolymer, the resulting prepolymer becomes hydrolyzable. In this case, however, the prepolymer produces an aromatic compound of high toxicity as a hydrolysis product, which poses a problem.

Compounds having an aromatic ring generally have problems of, for example, (a) possible carcinogenicity, (b) difficulty of being metabolized in a living body and (c) toxicity; therefore, it is desirable to use an aliphatic isocyanate and not to use an aromatic isocyanate. However, the aliphatic isocyanate has low reactivity and, in the reaction with a polyol or water, is unable to show a high reaction rate unless there is used, in combination, a catalyst of high toxicity, such as amine, organometal compound or the like.

The object of the present invention is to provide a surgical adhesive which is rapidly cured by the combination of an aliphatic isocyanate of low reactivity and a catalyst of low toxicity, whose cured material is desirably decomposable in a living body, and whose decomposition product has low toxicity.

The present inventors made study in order to obtain a surgical adhesive which gives low toxicity to a living body. As a result, the present inventors found that an aliphatic isocyanate-terminated urethane prepolymer having an isocyanate group at substantially all the molecular ends, obtained by adding a polyol having a particular molecular weight and a particular number of functional groups to an aliphatic diisocyanate, can be rapidly cured when reacted with water in the presence of a metal carboxylate of low toxicity. The finding has led to the completion of the present invention.

According to the present invention, there is provided a surgical adhesive comprising

(a) 100 parts by weight of an aliphatic isocyanate-terminated urethane prepolymer, and

(b) 0.1-5 parts by weight of a metal carboxylate as a curing catalyst.

As the aliphatic isocyanate-terminated urethane prepolymer (a) used in the surgical adhesive of the present invention, there is particularly preferred an aliphatic isocyanate-terminated urethane prepolymer containing no aromatic ring, obtained by adding an aliphatic diisocyanate to an aliphatic polyol.

The aliphatic polyol includes aliphatic polyols of bi- or tri-functionality such as polyether polyols [e.g. polyethylene oxide, polypropylene oxide, poly(ethylene oxidepropylene oxide) copolymer] obtained by adding an alkylene oxide (e.g. ethylene oxide, propylene oxide, butylene oxide) to a dihydroxy or trihydroxy aliphatic alcohol (e.g. ethylene glycol, propylene glycol, hexanetriol, glycerine); polyester polyols obtained by condensing an aliphatic dicarboxylic acid (e.g. oxalic acid, malonic acid, succinic acid) with an aliphatic diol (e.g. ethylene glycol, propylene glycol); polyester polyols obtained by subjecting a dihydroxy or trihydroxy aliphatic alcohol as mentioned above, a polyether polyol as mentioned above or a polyester polyol as mentioned above and a lactone (e.g. caprolactone, glycollide, lactide) to ring opening addition; polyester polyols obtained by condensing a dihydroxy or trihydroxy aliphatic alcohol as mentioned above, a polyether polyol as mentioned above or a polyester polyol as mentioned above, with a hydroxy aliphatic carboxylic acid (e.g. glycolic acid, lactic acid); and the like.

The aliphatic polyol desirably has a molecular weight of generally 200-5,000, preferably 400-3,000. When the aliphatic polyol has a molecular weight of less than 200, the prepolymer obtained by adding a diisocyanate to the aliphatic polyol contains the hydrophobic portion in a large proportion and accordingly has poor compatibility with water with which the prepolymer is to be reacted for curing, making it difficult for the curing reaction to proceed. In general, in homologue polymer compounds, a compound of lower molecule tends to have higher toxicity. When the aliphatic polyol has a molecular weight of more than 5,000, the resulting prepolymer contains the terminal isocyanate group in a small proportion and accordingly has low reactivity with water, making it difficult for the curing reaction to proceed.

An aliphatic polyol having an aliphatic ester structure in the main chain, for example, a polyester polyol is hydrolyzable. Therefore, the surgical adhesive obtained by using such a polyol, is decomposable in a living body. An aliphatic polyol having a glycolic acid residue or a lactic acid residue in the main chain, for example, a polyester polyol obtained by condensing a dihydroxy or trihydroxy alcohol with glycolic acid or lactic acid, is particularly preferable because it produces, by hydrolysis, glycolic acid or lactic acid which is nontoxic to a living body.

The aliphatic (alicyclic is also included) diisocyanate to be reacted with the aliphatic polyol, includes, for example, hexamethylene diisocyanate, isophorone diisocyanate, dicyclohexylmethane diisocyanate, methylcyclohexane diisocyanate and dimethylcyclohexane diisocyanate. Of these, 1,6-hexamethylene diisocyanate is particularly preferable because (a) the prepolymer produced therefrom has a primary isocyanate group at the terminals and accordingly is reactive, (') 1,6-hexamethylene diisocyanate is low-molecular and accordingly the resulting prepolymer contains the hydrophobic portion in a small proportion and has good miscibility with water with which the prepolymer is to be reacted, and consequently (c) the reaction between the prepolymer and water can be made smooth.

The addition of the aliphatic diisocyanate to the aliphatic polyol can be carried out in a urethane prepolymer method which is known per se. In this case, the ratio of the aliphatic polyol and aliphatic diisocyanate fed can be generally 1.4 or more in terms of NCO/OH ratio. When the ratio (NCO/OH ratio) is smaller than 1.4, the resulting prepolymer is high-molecular owing to chain growth, bringing about an increased viscosity and making difficult the mixing and stirring during curing. When the ratio is larger than 2, unreacted isocyanate monomer remains in the prepolymer. In this case, chain growth as a side reaction is unlikely to occur; however, it is necessary to remove the remaining isocyanate monomer from the prepolymer by distillation. If the isocyanate monomer is removed by distillation, the resulting prepolymer can be used, but such excessive use of isocyanate monomer is uneconomical. Therefore, a sufficient NCO/OH ratio is 2.2 or less.

The resulting urethane prepolymer can have a number-average molecular weight of generally 500-5,000m preferably 700-4,000.

The thus obtained aliphatic isocyanate-terminated urethane prepolymer can be used singly or in combination of two or more kinds. The curing of the prepolymer is made possible by (a) the reaction between the terminal aliphatic isocyanate group and water to form a terminal amine and (b) the reaction between this terminal amine and the terminal aliphatic isocyanate group of other prepolymer molecule to form a urea bond and bring about chain growth. It is possible to use, as a curing catalyst, a metal carboxylate. The metal carboxylate includes metal aliphatic carboxylates, for example, alkali metal or alkaline earth metal salts of aliphatic carboxylic acids (e.g. $C_{2-10}$ alkanoic acids such as acetic acid, propionic acid, butyric acid, valeric acid, caproic acid, capric acid, caprylic acid, 2-ethylhexyl acid and the like). Potassium or sodium aliphatic carboxylates are particularly preferable because they have low toxicity and give little effect to a living body.

The amount of the curing catalyst can be generally 0.1-5 parts by weight, preferably 1-3 parts by weight per 100 parts by weight of the prepolymer. When the amount is less than 0.1 part by weight, the curing reaction does not proceed rapidly. When the amount is more than 5 parts by weight, the curing reaction is too rapid and tends to form a nonuniform cured material.

In order to allow the curing reaction to proceed smoothly, it is possible to add to the surgical adhesive of the present invention an aliphatic polyol as mentioned above, having bi- or tri-functionality and a molecular weight of generally 200-3,000, particularly 400-3,000, for the purpose of viscosity adjustment, compatibility increase and crosslinking acceleration. The amount of the aliphatic polyol added is not critical but is generally 30 parts by weight or less per 100 parts by weight of the prepolymer.

It is also possible to add to the present surgical adhesive a foam stabilizer which is generally used in the urethane field. This makes it possible to obtain a cured material containing bubbles of uniform size.

The adhesive of the present invention can be used as a surgical adhesive in applications such as substitution for suturation using a suture, filling of lost portions, coating of damaged portions, prevention of adhesion of living tissue, hemostasis and the like.

In using the present adhesive in such applications, the addition of water to the present adhesive is not necessary when the living tissues to which the adhesive is applied contain water of an amount enough for the curing of the adhesive (generally, water of 70-500%, preferably 80-100% relative to the isocyanate equivalents of the adhesive). However, it is generally convenient to apply the present adhesive to an intended living body site after adding to the adhesive water of 70-500%, particularly 80-100% relative to the isocyanate equivalents of the adhesive depending upon the water amount present in the site. Particularly when the present adhesive is applied to an adherend containing substantially no water, it is necessary to add to the adhesive water of at least 70% relative to the isocyanate equivalents of the adhesive. The portion of water exceeding the isocyanate equivalents is not directly involved in the curing of the adhesive, but water of up to 500% is allowed. The presence of large excess of water exceeding 500% results in predominant formation of an amine-terminated prepolymer by the reaction of terminal isocyanate and water, and the reaction between said amine-terminated prepolymer and isocyanate-terminated prepolymer does not take place sufficiently; accordingly, a nonuniform cured material is formed easily. Therefore, in such a case, it is desirable to adjust water amount by, for example, wiping off water from the living tissue to which the adhesive is applied.

The application of the present adhesive to which an appropriate amount of water has been added, to an adherend, can be made, for example, by direct coating using a spatula or the like, spray coating, or injection using a syringe, a tube or the like.

The surgical adhesive of the present invention is characterized in that it comprises an aliphatic isocyanate-terminated urethane prepolymer and a metal carboxylate, contains no aromatic compound, low-molecular amine and organometal compound which have a toxicity problem, and is rapidly cured with water to form a cured material which in turn is hydrolyzed in a living body to form a hydrolysis product which can be metabolized. Accordingly, the present surgical adhesive is very safe to a living body and, when used as a medical material for surgery, can provide an improved technique in medical treatment.

The present invention is hereinafter described more specifically by way of Examples.

Preparation of urethane prepolymers

(A) 336.4 g of HID (hexamethylene diisocyanate manufactured by NIPPON POLYURETHANE INDUSTRY, CO., LTD.) was taken in a four-necked flask provided with a cooling tube, and heated to 80°C with stirring. Thereto was dropwise added 400 g of Carbowax #400 (a polyethylene glycol having an average molecular weight of 400, manufactured by Kokusan Chemical Limited) in 3 hours (the ratio of hexamethylene diisocyanate and polyethylene glycol fed was NCO/OH = 2). After the completion of the dropwise addition, stirring was continued for a further 6 hours at 80°C to obtain a "prepolymer A".

(B) A "prepolymer B" was obtained in the same manner as in the above (A) by using HDI and GL-3000 (a trifunctional polyol having a molecular weight of 3,000, manufactured by Sanyo Chemical Industries, Ltd.) at a ratio of NCO/OH = 2.

(C) A prepolymer having a glycolic acid residue was synthesized as follows. In a four-necked flask provided with a cooling tube were taken 114 g of glycolic acid (first class grade chemical, manufactured by Wako Pure Chemical Industries, Ltd.) and 200 g of Carbowax #400. They were heated with stirring at normal pressure for refluxing. With the progress of a reaction, the refluxing temperature increased, but the esterification reaction soon reached an equilibrium and the refluxing temperature became constant (120°C) in about 4 hours from the start of heating. Successively, the mixture was heated to 180°C to give rise to a reaction for 3 hours while reducing the pressure to 35 mmHg using an aspirator, to obtain a polyol having a glycolic acid residue in the main chain and an average molecular weight of 520 (high performance liquid chromatography was used for the measurement of the average molecular weight). 168 g of HDI was taken in another four-necked flask provided with a cooling tube and heated to 80°C with stirring. Thereto was dropwise added the whole amount of the above polyol in 3 hours. After the completion of the dropwise addition, the reaction was continued for a further 6 hours at 80°C to obtain a "prepolymer C".

(D) A prepolymer having a lactic acid residue was synthesized as follows in the same manner and same reaction conditions as in the above (C) except that dl-lactic acid was used in place of glycolic acid. That is, in a four-necked flask provided with a cooling tube were taken 135 g of dl-lactic acid (special grade chemical, manufactured by Kokusan Chemical Limited) and 200 g of Carbowax #400. They were heated with stirring at normal pressure for refluxing. With the progress of a reaction, the refluxing temperature increased, but the esterification reaction soon reached an equilibrium and the refluxing temperature became constant (140°C) in about 4 hours from the start of heating. Successively, the mixture was heated to 180°C to give rise to a reaction for 3 hours while reducing the pressure to 35 mmHg using an aspirator, to obtain a polyol having a lactic acid residue in the main chain and an average molecular weight of 580. 168 g of HDI was taken in another four-necked flask provided with a cooling tube and heated to 80°C with stirring. Thereto was dropwise added the whole amount of the above polyol in 3 hours. After the completion of the dropwise addition, the reaction was continued for a further 6 hours at 80°C to obtain a "prepolymer D".

Preparation of catalysts

1 part of potassium octanoate was dissolved in 1 part of TG-400 (a polyether polyol manufactured by Mitsui Nisso K.K.). The resulting solution was used as a "catalyst M".

1 part of sodium acetate was dissolved in 1 part of water. The resulting solution was used as a "catalyst N".

Examples 1-3 and Comparative Example 1

The aliphatic isocyanate-terminated prepolymer obtained above was mixed with water and a catalyst to give rise to curing. The curing was carried out at room temperature (25°C) in all cases.

In Examples 1-3, 10 g of the prepolymer A was taken in a cup; thereto were added the catalyst M, water and a silicone oil (L-5430 manufactured by Nippon Unicar Co., Ltd.) as a foam stabilizer, in amounts as shown in Table 1; the resulting mixture was stirred for 10 seconds at 2,000 rpm using a cage-shaped mixer; then, the mixture was filled between two glass plates containing a 1-mm spacer placed between them, to give rise to curing to obtain a foamed sheet-shaped sample. The amount of water added was equal to the isocyanate equivalents of the mixture in Example 1, 450% of the isocyanate equivalents in Example 2 and 80% of the isocyanate equivalents in Example 3.

In Table 1, "curing time" refers to a time required for a foamed material obtained by a curing reaction to become tack-free. The tensile test of a cured material was carried out at a tensile speed of 10 cm/min.

In Comparative Example 1, to 10 g of the prepolymer A were added 0.6 g of ethylene glycol and 0.5 g of the catalyst M; and curing by urethane bond formation was carried out under the same conditions as in Example 1. The results are shown in Table 1.

Table 1

| | | Composition | | | | | | Properties of cured material | | | |
|---|---|---|---|---|---|---|---|---|---|---|---|
| | | Pre-polymer A (g) | water (g) | Ethylene glycol (g) | Polyol (g) | Potassium octanoate (g) (catalyst M) | Silicone L-5430 (g) | Curing time (sec) | Breaking strength (kg/cm$^2$) | Breaking Exten-sion (%) | Density (g/cm$^3$) |
| Example | 1 | 10 | 0.24 | – | 0.05 | 0.05 | 0.5 | 120 | 17.8 | 104 | 0.43 |
| | 2 | 10 | 1.08 | – | 0.05 | 0.05 | 0.5 | 80 | 21.0 | 50 | 0.57 |
| | 3 | 10 | 0.20 | – | 0.25 | 0.25 | 0.5 | 30 | 34.3 | 208 | 0.62 |
| Compara-tive Example | 1 | 10 | – | 0.9 | 0.25 | 0.25 | 0.5 | Cured material is fragile | | | |

Examples 4-8

Examples 4-6 are curing examples for compositions using different prepolymers made from different polyol components. That is, 10 g of the prepolymer B, C or D was taken in a cup; thereto were added the catalyst M and water in amounts shown in Table 2; the resulting mixture was stirred for 10 seconds at 2,000 rpm using a cage-shaped mixer, to give rise to curing. The prepolymers used in Examples 5 and 6 contained a glycolic acid residue or a lactic acid residue, and their cured materials are hydrolyzable. Therefore, there cured materials were subjected to a hydrolysis test. That is a cube having a side of 3 cm was cut out from each cured material and allowed to stand in 200 cc of water kept at 36°C to examine the days required for decomposition. Here, "decomposition" refers to that the cured material in the water is converted from the original shape to flaky small pieces by hydrolysis and becomes a suspension. The results are shown in Table 2.

Example 7 is a curing example for a composition using a different catalyst component. That is, 10 g of the prepolymer A was taken in a cup; thereto was added the catalyst N in an amount shown in Table 2; the resulting mixture was stirred for 10 seconds at 2,000 rpm using a cage-shaped mixer, to give rise to curing. The results are shown in Table 2.

In Example 8, only a catalyst was added to a prepolymer and no water was added; beef was used as a substitute for living tissue; and curing was carried out on the beef. That is, the catalyst M was added to 10 g of the prepolymer A in an amount shown in Table 2; and the mixture was stirred for 10 seconds at 2,000 rpm using a cage-shaped mixer. The mixture was coated on a beef whose surface water had been removed by gentle wiping with absorbent cotton and which had been heated to 36°C, in a thickness of 0.3 mm by means of a spatula, and the mixture was cured with water in the beef. The results are shown in Table 2.

Table 2

| Example | Pre-polymer 10g | Water (g) | Polyol (g) | Potassium octanoate (catalyst M) | Sodium acetate (catalyst N) | Curing time | Days required for decomposition |
|---|---|---|---|---|---|---|---|
| | | | | Composition | | | |
| 4 | B | 0.08* | 0.25 | 0.25 | – | 3 | – |
| 5 | C | 0.21* | 0.25 | 0.25 | – | 2 | 30 |
| 6 | D | 0.20* | 0.25 | 0.25 | – | 2 | 40 |
| 7 | A | 0.25* | – | – | 0.25 | 5 | – |
| 8 | A | Water in beef | 0.25 | 0.25 | – | 6 | – |

* Equal to isocyanate equivalents.

## Claims

1. A surgical adhesive comprising
(a) 100 parts by weight of an aliphatic isocyanate-terminated urethane prepolymer, and
(b) 0.1-5 parts by weight of a metal carboxylate as a curing catalyst.

2. The surgical adhesive of claim 1, wherein the aliphatic isocyanate-terminated urethane prepolymer is obtained by adding an aliphatic diisocyanate to an aliphatic polyol and contains no aromatic ring.

3. The surgical adhesive of claim 2, wherein the aliphatic polyol has a molecular weight of 200-5,000 and a bi- or tri-functionality.

4. The surgical adhesive of claim 2, wherein the aliphatic polyol is a polyester polyol.

5. The surgical adhesive of claim 2, wherein the aliphatic polyol is a polyester polyol obtained by condensing a dihydroxy or trihydroxy aliphatic alcohol with glycolic acid or lactic acid.

6. The surgical adhesive of claim 2, wherein the aliphatic diisocyanate is 1,6-hexamethylene diisocyanate.

7. The surgical adhesive of claim 1, wherein the aliphatic isocyanate-terminated urethane prepolymer has number-average molecular weight of 700-4,000.

8. The surgical adhesive of claim 1, wherein the metal carboxylate is a potassium aliphatic carboxylate or a sodium aliphatic carboxylate.

9. The surgical adhesive of claim 1, which comprises the metal carboxylate in an amount of 1-3 parts by weight per 100 parts by weight of the aliphatic isocyanate-terminated urethane prepolymer.

10. The surgical adhesive of claim 1, which further comprises an aliphatic polyol having a molecular weight of 200-3,000 and bi- or tri-functionality.

11. A method for applying the surgical adhesive of claim 1, which comprises applying the surgical adhesive to an adherend in the presence of water of 70-500% relative to the isocyanate equivalents of the surgical adhesive.